# EUROPÄISCHE PATENTSCHRIFT

(11) **EP 1 013 294 B1**
(45) Veröffentlichungstag und Bekanntmachung des Hinweises auf die Patenterteilung: **18.04.2007**
(21) Anmeldenummer: 99811060.5
(22) Anmeldetag: 17.11.1999
(51) Int. Cl.: A61M 1/10, H02K 7/09

(54) **Diagonalflusspumpe**
Diagonal flux pump
Pompe à flux diagonal

(30) Priorität: 16.12.1998 EP 98811236
(43) Veröffentlichungstag der Anmeldung: 28.06.2000
(73) Patentinhaber: Levitronix LLC, Waltham, MA 02451 (US)
(72) Erfinder: Schöb, Reto, Dr., 8604 Volketswil (CH)
(74) Vertreter: Sulzer Management AG

(56) Entgegenhaltungen:
- EP-A- 0 425 257
- EP-A- 0 810 374
- WO-A-93/20860
- WO-A-97/42414
- WO-A-98/11650
- DE-A- 19 626 224

## Beschreibung

Die Erfindung betrifft eine Diagonalflusspumpe gemäss dem Oberbegriff des unabhängigen Patentanspruchs.

Pumpen, bei denen der Rotor magnetisch - also berührungslos - gelagert und angetrieben wird, sind in verschiedenen Ausführungsformen bekannt. Sie kommen insbesondere in solchen Gebieten zum Einsatz, bei denen ein Kontakt des zu fördernden Mediums mit mechanischen Lagern vermieden werden soll. Ein typisches Anwendungsbeispiel hierfür ist eine Blutpumpe, bei welcher eine Kontamination des zu fördernden Blutes vermieden werden soll. Besonders verbreitet sind dabei Zentrifugalpumpen und Axialpumpen.

Das Dokument WO 98/11650 beschreibt sowohl Axial- als auch Zentrifugalpumpen mit magnetisch gelagertem und angetriebenem Rotor.

Bei der Zentrifugalpumpe wird das zu fördernde Medium, also z.B. das Blut, durch einen in Bezug auf das Pumpenrad axial angeordneten Einlass angesaugt. Mit Hilfe des Pumpenrads und dessen Pumpenschaufeln wird das Medium dann radial oder tangential zum dort angeordneten Auslass gefördert. Zentrifugalpumpen haben einen verhältnismässig grossen Aussendurchmesser, der für VAD-Anwendungen (Ventricle Assist Devices), bei welchen typischerweise ein Druck in der Grössenordnung von etwa 150 hPa und ein Durchfluss von etwa 7 Liter/min gefordert ist, im Bereich von etwa 30-50 mm liegt. Für Zentrifugalpumpen mit Rotoren, die einen Aussendurchmesser aufweisen, der in dem genannten Bereich liegt, liegt die spezifische Drehzahl n_{q} im Bereich von n_{q}<40. Die spezifische Drehzahl ist dabei charakteristisch für einen Bereich, in welchem die Pumpe bezüglich ihres Wirkungsgrads am effektivsten betrieben wird. Sie ist typischerweise spezifiziert als n_{q} = n x Q^{1/2} / h^{3/4}, wobei n die Drehzahl der Pumpe bedeutet [dimensionslos], Q den Durchfluss [m³/s] und h den Druck [m], wobei man sich die Einheit für den Druck hier so vorstellen kann, dass sie die Höhe einer für einen solchen Druck erforderlichen Wassersäule bezeichnet.

Bei der Axialpumpe wird das zu fördernde Medium, also z.B. das Blut, durch einen in Bezug auf das Pumpenrad axial angeordneten Einlass angesaugt und mit Hilfe der Pumpenschaufeln zu einem ebenfalls axial in Bezug auf das Pumpenrad angeordneten Auslass gefördert, der allerdings auf der anderen Seite des Pumpenrads angeordnet ist. Grundsätzlich sind hier spezifische Drehzahlen n_{q} im Bereich von 150-300 Umdrehungen pro Minute von der Auslegung der Pumpe her möglich. Allerdings ist dabei zu bedenken, dass bei der Axialpumpe die volle Druckdifferenz zwischen dem Druck auf der Einlasseite und dem Druck auf der Auslasseite über dem Pumpenrad liegt, andererseits die Axiallagerkraft beschränkt ist, insbesondere bei passiven axialen Lagerungen.

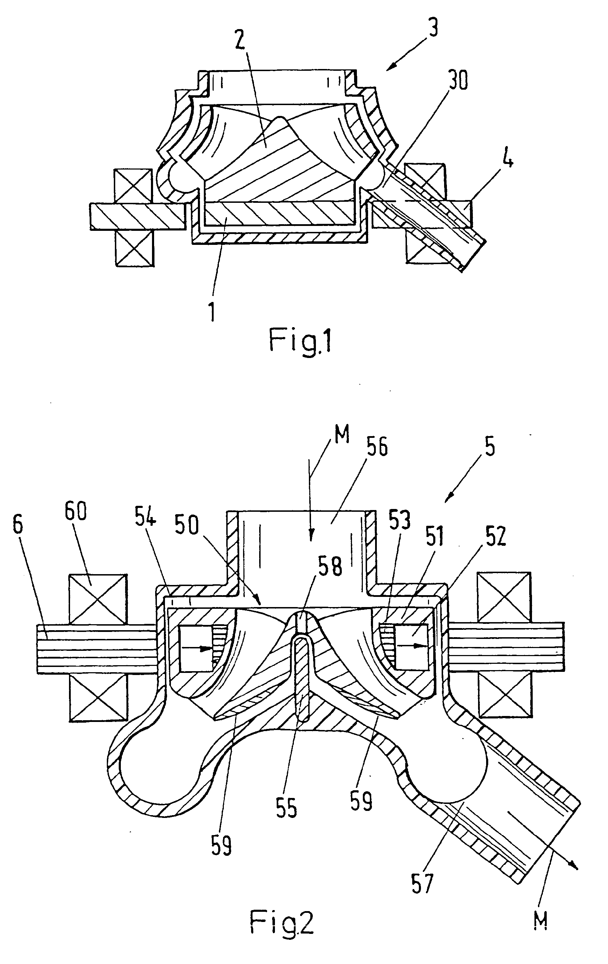

Als guter Kompromiss zwischen diesen beiden Pumpentypen hat sich die Diagonalflusspumpe erwiesen, bei welcher die spezifische Drehzahl typischerweise im Bereich von 40<n_{q}<150 liegt. Allerdings weist auch die Diagonalflusspumpe Nachteile auf. So werden zum Beispiel die Abmessungen der gesamten Rotoranordnung in axialer Richtung relativ gross, wenn das Pumpenrad 2 auf den angetriebenen Rotor 1 aufgesetzt wird (siehe Fig. 1). Die Hauptträgheitsmomente liegen dann nahe beieinander und führen so zu einer Instabilität einer derartigen Rotoranordnung. Ausserdem ist es schwierig, den in diagonaler Richtung versetzten Auslass 30 der Pumpe 3 am Stator 4 vorbeizuführen.

Es ist daher eine Aufgabe der Erfindung, eine Diagonalflusspumpe vorzuschlagen, welche die vorstehend genannten Nachteile nicht aufweist, die also insbesondere einen Rotor aufweist, welcher mit Hilfe einer magnetischen Lagerung sehr stabil gelagert und angetrieben werden kann. Gleichzeitig soll es keinerlei Schwierigkeiten bereiten, den Auslass der Diagonalflusspumpe am Stator bzw. an den auf dem Stator befindlichen Wicklungen vorbeizuführen.

Diese Aufgabe wird durch eine Diagonalflusspumpe gelöst, wie sie durch die Merkmale des unabhängigen Patentanspruchs charakterisiert ist. Die erfindungsgemässe Diagonalflusspumpe umfasst also einen Rotor und einen radial angeordneten Lager- und Antriebsstator, mit welchem der Rotor magnetisch gelagert und angetrieben wird, sowie ein Pumpenrad, mit dessen Hilfe ein zu förderndes Medium vom Einlass der Pumpe zu deren Auslass gefördert wird. Der vom Lager- und Antriebsstator gelagerte und angetriebene Rotor und das Pumpenrad sind baulich ineinander integriert und bilden einen einzigen Pumpenrotor mit Pumpenschaufeln, wobei der Rotor innerhalb der axialen Abmessungen der Pumpenschaufeln liegt. Dadurch wird einerseits vermieden, dass mehrere Hauptträgheitmomente nahe beieinander liegen und eine Instabilität des Rotors bewirken können, sondern vielmehr erhält man auf diese Weise einen kompakten, axial und gegen Verkippung stabil magnetisch lagerbaren, Pumpenrotor. Durch das Ineinanderintegrieren von Pumpenrad und Rotor zu einem einzigen Pumpenrotor kann auch der Auslass der Diagonalflusspumpe so angeordnet werden, dass es keine Schwierigkeiten bereitet, den Auslass der Pumpe am Stator vorbeizuführen.

Bei einem vorteilhaften Ausführungsbeispiel umfasst der Pumpenrotor Permanentmagnete, welche in den Pumpenschaufeln des Pumpenrotors angeordnet sind. Permanentmagnetisch erregte Antriebe zeichnen sich dadurch aus, dass der von den Permanentmagneten aufgebrachte magnetische Fluss nicht durch elektrische Ströme erzeugt werden muss, was energetisch und auch bezüglich der von Elektromagneten erzeugten, unerwünschten Wärme günstiger ist.

Bei einem weiteren vorteilhaften Ausführungsbeispiel sind der Lager- und Antriebsstator und der Pumpenrotor so ausgebildet und angeordnet, dass der Lager- und Antriebsstator die Lagerung und den Antrieb des Pumpenrotors in der Ebene des Lager- und Antriebsstators bewirkt. Dies ermöglicht einerseits eine kompakte Bauweise der Pumpe und vereinfacht andererseits die Art und Weise, wie der Pumpenrotor gelagert werden kann.

Bei einem weiteren vorteilhaften Ausführungsbeispiel bzw. bei einer Weiterbildung sind der Lager- und Antriebsstator und der Pumpenrotor so ausgebildet und angeordnet, dass der Lager- und Antriebsstator in der Ebene des Lager- und Antriebsstators ein Radiallager für den Pumpenrotor bildet, wobei der Pumpenrotor in axialer Richtung und bezüglich Verkippung passiv magnetisch gelagert ist. Dies bedingt eine einfache Art und Weise der Lagerung des Pumpenrotors, bei welchem nämlich der Aufwand für die aktive Regelung dreier Freiheitsgrade entfallen kann, nämlich der Aufwand für die Regelung der axialen Lagerung des Pumpenrotors sowie der Aufwand für die Regelung der Verkippung des Rotors um die beiden Kippachsen.

Bei einem weiteren vorteilhaften Ausführungsbeispiel sind der Einlass und der Auslass der Pumpe auf verschiedenen Seiten, z.B. oberhalb und unterhalb, des Lager- und Antriebbsstators angeordnet. Dadurch kommt es zu keinerlei räumlichen Konflikten des Einlasses bzw. Auslasses der Pumpe mit den Statorwicklungen.

Bei einem weiteren vorteilhaften Ausführungsbeispiel ist der Stator ausserhalb des Pumpengehäuses angeordnet und das Pumpengehäuse ist aus einem unmagnetischen Material hergestellt. Dies ermöglicht, dass das zu fördernde Medium mit dem Stator nicht in Kontakt kommen kann, sodass diesbezüglich keine besonderen Massnahmen zur Vermeidung von Korrosion etc. getroffen werden müssen. Da der Stator andererseits das für die Lagerung und den Antrieb des Pumpenrotors erforderliche magnetische Feld erzeugt, muss das Pumpengehäuse aus einem unmagnetischen Material, z.B. einem Kunststoff, hergestellt sein.

Bei einem weiteren vorteilhaften Ausführungsbeispiel der erfindungsgemässen Diagonalflusspumpe ist ein Notlauflager für den Pumpenrotor vorgesehen, welches für den Fall, dass die magnetische Lagerung des Rotors einmal ausfällt, eine Beschädigung des Rotors verhindert.

Ein weiteres vorteilhaftes Ausführungsbeispiel der erfindungsgemässen Diagonalflusspumpe zeichnet sich dadurch aus, dass der Pumpenrotor eine Entlastungs- und Auswaschbohrung aufweist. Diese kann einerseits eine Entlastung des Pumpenrotors bewirken im Hinblick auf die Axialkräfte, die auf den Rotor wirken, sie bewirkt aber auch, dass auslasseitig im zentralen Bereich des Pumpenrotors stets eine gute Durchspülung gewährleistet ist. Dies ist insbesondere bei Blut als zu förderndem Medium wichtig, weil dadurch Anlagerungen im zentralen Bereich vermieden werden können.

Ein weiteres vorteilhaftes Ausführungsbeispiel der erfindungsgemässen Diagonalflusspumpe zeichnet sich dadurch aus, dass der Pumpenrotor mit Rudimentärpumpenschaufeln versehen ist, welche auslasseitig am Pumpenrotor angeordnet sind zum Fördern von Medium aus einem zentralen Bereich des Pumpenrotors nach aussen hin. Dies bewirkt eine Absenkung des Drucks auf der Auslasseite und damit eine Entlastung des Pumpenrotors im Hinblick auf Axialkräfte, die aufgrund der Druckdifferenz zwischen Einlass- und Auslasseite auf den Pumpenrotor wirken. Weitere vorteilhafte Ausgestaltungen ergeben sich aus der nachfolgenden Beschreibung.

Im folgenden wird die Erfindung anhand der Zeichnung näher erläutert. Es zeigen in schematischer Darstellung und im Schnitt:
- Fig. 1: eine Diagonalflusspumpe mit einem magnetisch gelagerten Rotor und einem Pumpenrad zur Verdeutlichung der bei derartigen Pumpen auftretenden Probleme
und
- Fig. 2: ein Ausführungsbeispiel einer erfindungsgemässen Diagonalflusspumpe, bei welcher der Rotor und das Pumpenrad ineinander intergriert sind und einen einzigen Pumpenrotor bilden.

Fig. 2 zeigt ein Ausführungsbeispiel einer erfindungsgemässen Diagonalflusspumpe 5. Man erkennt, dass der (Motor-)Rotor und das Pumpenrad ineinander integriert sind und einen einzigen Pumpenrotor 50 bilden. Pumpenrad und (Motor-)Rotor sind hier also identisch, d.h. sind ein- und dasselbe Bauteil, nämlich der Pumpenrotor 50. Die Ebene des Pumpenrads und die Ebene des (Motor-)Rotors fallen somit in der Ebene des Pumpenrotors 50 zusammen. In dem Pumpenrotor 50, genauer gesagt in den Schaufeln 51 des Pumpenrotors 50, sind Permamentmagnete 52 und Rückschlusseisen 53 angeordnet. Ferner erkennt man einen Stator 6 mit (Stator-)Wicklungen 60, die mit den Permanentmagneten 52 zusammenwirken und die in der Ebene des Stators 6 sowohl die berührungsfreie magnetische Lagerung als auch den Antrieb des (etwa scheibenförmigen) Pumpenrotors 50 in bekannter Weise bewirken. Dadurch wird eine kompakte Bauweise der Pumpe ermöglicht. Die axiale Lagerung wie auch die Stabilisierung des Pumpenrotors 50 gegen Verkippung wird hier passiv bewirkt, nämlich durch ein Radiallager, welches durch den Stator 6 und dessen (Stator-)wicklungen 60 gebildet wird und mit den Permanentmagneten 52 des Pumpenrotors 50 zusammenwirkt.

Zwischen dem Stator 6 und dem Pumpenrotor 50 ist ein Pumpengehäuse 54 angeordnet, welches aus einem unmagnetischen Material, z.B. einem Kunststoff, hergestellt ist. Der Stator 6 kommt also nicht in Berührung mit dem zu fördernden Medium (z.B. Blut), sodass keinerlei vorbeugende Massnahmen z.B. gegen Korrosion des Stators 6 getroffen werden müssen. Hingegen kommt der Pumpenrotor 50 zwangsläufig in Kontakt mit dem zu fördernden Medium, sodass bei einer mehrfachen Verwendung entsprechende Massnahmen zu treffen sind (z.B. kann der Pumpenrotor mit einem Überzug versehen werden) oder die Pumpe (mit Ausnahme des Stators) als Einmalpumpe (Wegwerfpumpe) ausgeführt ist.

Ferner erkennt man noch einen Stift 55, welcher im Pumpengehäuse 54 fest angeordnet ist. Dieser Stift 55 bildet ein Notlauflager für den Pumpenrotor 50 für den Fall, dass die magnetische Lagerung des Pumpenrotors 50 ausfällt. Dadurch wird in solchen Fällen eine Beschädigung von Teilen der Pumpe vermieden. Bei einem Ausfall der magnetischen Lagerung (also z.B. bei einem Ausfall der elektrischen Versorgung der Wicklungen 60) wird der Pumpenrotor von den Permanentmagneten 52 in Richtung auf eine der Seitenwände des Pumpengehäuses 54 zu gezogen (dahinter befinden sich ja die Statorbleche), je nachdem, welcher der Permanentmagnete stärker ist. Der Stift 55 verhindert bei einer solchen Seitwärtsbewegung des Pumpenrotors 50, dass der Pumpenrotor 50 an dem Pumpengehäuse anschlägt und möglicherweise Teile der Pumpe beschädigt werden.

Im Pumpenrotor 50 ist an der dem Stift 55 gegenüberliegenden Stelle des Pumpenrotors 50 optional eine Entlastungs- und Auswaschbohrung 58 (bzw. Ausgleichsbohrung) vorgesehen. Diese Entlastungs- und Auswaschbohrung 58 hat folgende Funktion: Wenn man sich vorstellt, dass auf der Auslasseite (also unterhalb des Pumpenrotors 50) ein höherer Druck herrscht als auf der Einlasseite (also oberhalb des Pumpenrotors 50), so wirkt auf den Pumpenrotor 50 eine in Richtung der Einlasseite wirkende Axialkraft. Der Pumpenrotor 50 muss daher mit Hilfe der axial passiven magnetischen Lagerung (Radiallager) axial ausreichend stabil gelagert sein (dies gilt übrigens auch bezüglich der Verkippung des Rotors). Die Entlastungs- und Auswaschbohrung 58 hat nun zur Folge, dass die Druckdifferenz zwischen Einlass- und Auslasseite verringert wird und damit auch die auf den Pumpenrotor 50 wirkende Axialkraft, weil ja zu förderndes Medium M aufgrund des höheren Drucks von der Auslasseite durch die Entlastungs- und Auswaschbohrung 58 zurück zur Einlasseite strömen kann. Andererseits ist es auch so, dass durch die Entlastungs- und Auswaschbohrung 58 gewährleistet ist, dass der Bereich um den Stift 55 herum stets durchspült ist, sodass es dort nicht zu Anlagerungen kommen kann, was insbesondere bei Blut als zu förderndem Medium M wichtig ist.

Weiterhin können an dem Pumpenrotor 50 im unteren Bereich - also auslasseitig - optional noch sogenannte Ruidmentärpumpenschaufeln 59 vorgesehen sein. Die Rudimentärpumpenschaufeln 59 haben die Funktion, das zu fördernde Medium M aus dem zentralen Bereich, also aus dem Bereich um den Stift 55 herum, nach aussen zu fördern. Dies führt zu einem geringeren Druck auf der Auslasseite und damit zu einer geringeren Druckdifferenz, wodurch die Stabilität der passiven axialen Lagerung des Pumpenrotors 50 erhöht wird. Jedoch wird jetzt zu förderndes Medium M in Richtung von der Einlasseite zur Auslasseite hin durch die Entlastungs- und Auswaschbohrung 58 gesaugt (Staubsaugerprinzip), die hier nur noch als Auswaschbohrung fungiert, aber gleichwohl Sorge dafür trägt, dass der Bereich um den Stift 55 herum stets gut durchspült wird (es wird ja Medium M durch die Bohrung 58 angesaugt) und keine Anlagerungen am Stift 55 erfolgen können.

Durch das Ineinanderintegrieren des Rotors und des Pumpenrads, welche bei der Diagonalflusspumpe gemäss Fig. 1 aufeinander aufgesetzt sind, zu einem (mehr oder weniger scheibenförmigen) Pumpenrotor 50 wird erreicht, dass eine stabile magnetische Lagerung des Pumpenrotors 50 möglich ist, weil die anhand von Fig. 1 erläuterte Instabilität der Rotoranordnung, die von den nahe beieinander liegenden Hauptträgheitsmomenten von dem aufeinandergesetzten Rotor und dem Pumpenrad herrührt, bei der erfindungsgemässen Diagonalflusspumpe 5 nicht mehr vorhanden ist. Gleichzeitig wird durch das Ineinanderintegrieren von Rotor und Pumpenrad zu dem Pumpenrotor 50 bewirkt, dass der Auslass 57 der Pumpe in keinen räumlichen Konflikt mehr mit dem Stator 6 bzw. den Statorwicklungen 60 kommt, weil der Einlass 56 der Pumpe und der Auslass 57 problemlos auf verschiedenen Seiten des Stators 6 (hier: oberhalb und unterhalb) angeordnet werden können. Die Pfeile M bezeichnen das zu fördernde Medium, welches beim Betrieb der Diagonalflusspumpe 5 mit Hilfe des Pumpenrotors 50 vom Einlass 56 der Pumpe zu deren Auslass 57 gefördert wird.

## Patentansprüche

1. Diagonalflusspumpe (5) mit einem Rotor (50) und einem radial angeordneten Lager- und Antriebsstator (6,60), mit welchem der Rotor magnetisch gelagert und angetrieben wird, sowie mit einem Pumpenrad, mit dessen Hilfe ein zu förderndes Medium vom Einlass der Pumpe zu deren Auslass gefördert wird, wobei der vom Lager- und Antriebsstator (6,60) gelagerte und angetriebene Rotor und das Pumpenrad baulich ineinander integriert sind und einen einzigen Pumpenrotor (50) mit Pumpenschaufeln (51) bilden, **dadurch gekennzeichnet, dass** der Rotor (50) innerhalb der axialen Abmessungen der Pumpenschaufeln liegt.

2. Diagonalflusspumpe nach Anspruch 1, **dadurch gekennzeichnet, dass** der Pumpenrotor Permanentmagnete (52) umfasst, welche in den Pumpenschaufeln (51) des Pumpenrotors (50) angeordnet sind.

3. Diagonalflusspumpe nach einem der vorangehenden Ansprüche, **dadurch gekennzeichnet, dass** der Lager- und Antriebsstator (6) und der Pumpenrotor (50) so ausgebildet und angeordnet sind, dass der Lager- und Antriebsstator (6) die Lagerung und den Antrieb des Pumpenrotors (50) in der Ebene des Lager- und Antriebsstators (6) bewirkt.

4. Diagonalflusspumpe nach einem der vorangehenden Ansprüche, **dadurch gekennzeichnet, dass** der Lager- und Antriebsstator (6) und der Pumpenrotor (50) so ausgebildet und angeordnet sind, dass der Lager- und Antriebsstator (6) in der Ebene des Lager- und Antriebsstators (6) ein Radiallager für den Pumpenrotor (50) bildet und der Pumpenrotor (50) in axialer Richtung und bezüglich Verkippung passiv magnetisch gelagert ist.

5. Diagonalflusspumpe nach einem der vorangehenden Ansprüche, **dadurch gekennzeichnet, dass** der Einlass (56) und der Auslass (57) der Pumpe auf verschiedenen Seiten, z.B. oberhalb und unterhalb, des Lager- und Antriebbsstators (6) angeordnet sind.

6. Diagonalflusspumpe nach einem der vorangehenden Ansprüche, **dadurch gekennzeichnet, dass** der Stator (6,60) ausserhalb des Pumpengehäuses (54) angeordnet ist und das Pumpengehäuse (54) aus einem unmagnetischen Material hergestellt ist.

7. Diagonalflusspumpe nach einem der vorangehenden Ansprüche, **dadurch gekennzeichnet, dass** ein Notlauflager (55) für den Pumpenrotor (50) vorgesehen ist.

8. Diagonalflusspumpe nach einem der vorangehenden Ansprüche, **dadurch gekennzeichnet, dass** der Pumpenrotor (50) eine Entlastungs- und Auswaschbohrung (58) aufweist.

9. Diagonalflusspumpe nach einem der vorangehenden Ansprüche, **dadurch gekennzeichnet, dass** der Pumpenrotor (50) mit Rudimentärpumpenschaufeln (59) versehen ist, welche auslasseitig am Pumpenrotor (50) angeordnet sind zum Fördern von Medium aus einem zentralen Bereich des Pumpenrotors (50) nach aussen hin.

## Claims

1. A diagonal flow pump (5) including a rotor (50) and a radially arranged bearing stator and drive stator (6, 60) by means of which the rotor is magnetically journalled and driven, and including a pump wheel with the help of which a medium to be forwarded is forwarded from the inlet of the pump to its outlet, wherein the rotor, which is journalled and driven by the bearing stator and drive stator (6, 60), and the pump wheel are constructionally integrated into one another and form a single pump rotor (50) with pump vanes, **characterised in that** the rotor (50) lies within the axial dimensions of the pump vanes.

2. A diagonal flow pump in accordance with claim 1, **characterised in that** the pump rotor includes permanent magnets (52) which are arranged in the pump vanes (51) of the pump rotor (50).

3. A diagonal flow pump in accordance with any one of the preceding claims, **characterised in that** the bearing stator and drive stator (6) and the pump rotor (50) are so designed and arranged that the bearing stator and drive stator (6) brings about the journalling and the driving of the pump rotor (50) in the plane of the bearing stator and drive stator (6).

4. A diagonal flow pump in accordance with any one of the preceding claims, **characterised in that** the bearing stator and drive stator (6) and the pump rotor (50) are so designed and arranged that the bearing stator and drive stator (6) form a radial bearing for the pump rotor (50) in the plane of the bearing stator and drive stator (6) and the pump rotor (50) is passively magnetically journalled in the axial direction and with respect to tilting.

5. A diagonal flow pump in accordance with any one of the preceding claims, **characterised in that** the inlet (56) and the outlet (57) of the pump are arranged on different sides of the bearing stator and drive stator (6), for example above and below the bearing stator and drive stator (6).

6. A diagonal flow pump in accordance with any one of the preceding claims, **characterised in that** the stator (6, 60) is arranged outside the pump housing (54) and the pump housing (54) is manufactured of a non-magnetic material.

7. A diagonal flow pump in accordance with any one of the preceding claims, **characterised in that** an emergency bearing (55) is provided for the pump rotor (50).

8. A diagonal flow pump in accordance with any one of the preceding claims, **characterised in that** the pump rotor (50) has a relief and flushing bore (58).

9. A diagonal flow pump in accordance with any one of the preceding claims, **characterised in that** the pump rotor (50) is provided with rudimentary pump vanes (59) which are arranged at the outlet side at the pump rotor (50) for the forwarding of medium outwardly from a central region of the pump rotor (50).

## Revendications

1. Pompe à flux diagonal (5) avec un rotor (50) et un stator de palier et d'entraînement (6,60) disposé radialement, au moyen duquel le rotor est logé et entraîné magnétiquement, et avec une roue de pompe à l'aide de laquelle un milieu à convoyer est convoyé de l'entrée de la pompe à sa sortie, où le rotor logé et entraîné par le stator de palier et d'entraînement (6, 60) et la roue de pompe sont intégrés l'un dans l'autre de par la construction et forment un seul rotor de pompe (50) avec des palettes de pompe (51), **caractérisée en ce que** le rotor (50) se situe à l'intérieur des dimensions axiales des palettes de pompe.

2. Pompe à flux diagonal selon la revendication 1, et **caractérisée en ce que** le rotor de pompe comprend des aimants permanents (52) qui sont disposés dans les palettes de pompe (51) du rotor de pompe (50).

3. Pompe à flux diagonal selon l'une des revendications précédentes, **caractérisée en ce que** le stator de palier et d'entraînement (6) et le rotor de pompe (50) sont réalisés et disposés de façon que le stator de palier et d'entraînement (6) provoque le logement et l'entraînement du rotor de pompe (50) dans le plan du stator de palier et d'entraînement (6).

4. Pompe à flux diagonal selon l'une des revendications précédentes, **caractérisée en ce que** le stator de palier et d'entraînement (6) et le rotor de pompe (50) sont réalisés et disposés de façon que le stator de palier et d'entraînement (6) forme dans le plan du stator de palier et d'entraînement (6) un palier radial pour le rotor de pompe (50), et que le rotor de pompe (50) soit logé dans la direction axiale et relativement à un basculement d'une manière magnétique passive.

5. Pompe à flux diagonal selon l'une des revendications précédentes, **caractérisée en ce que** l'entrée (56) et la sortie (57) de la pompe sont disposées sur des côtés différents, par exemple au-dessus et en dessous du stator de palier et d'entraînement (6).

6. Pompe à flux diagonal selon l'une des revendications précédentes, **caractérisée en ce que** le stator (6, 60) est disposé à l'extérieur du boîtier de pompe (54), et **en ce que** le boîtier de pompe (54) est réalisé en un matériau non-magnétique.

7. Pompe à flux diagonal selon l'une des revendications précédentes, **caractérisée en ce qu'**il est prévu un palier de roulement de secours (55) pour le rotor de pompe (50).

8. Pompe à flux diagonal selon l'une des revendications précédentes, **caractérisée en ce que** le rotor de pompe (50) présente un perçage de décharge et de lavage (58).

9. Pompe à flux diagonal selon l'une des revendications précédentes, **caractérisée en ce que** le rotor de pompe (50) est pourvu de palettes de pompe rudimentaires (59) qui sont disposées, côté sortie, au rotor de pompe (50) pour le convoyage d'un milieu d'une zone centrale du rotor de pompe (50) vers l'extérieur.
